# EUROPÄISCHE PATENTSCHRIFT

(11) **EP 1 685 097 B1**
(45) Veröffentlichungstag und Bekanntmachung des Hinweises auf die Patenterteilung: **13.02.2008**
(21) Anmeldenummer: 04797536.2
(22) Anmeldetag: 03.11.2004
(51) Int. Cl.: C07C 381/00

(54) **VERFAHREN ZUR HERSTELLUNG VON 4-PENTAFLUORSULFANYL-BENZOYLGUANIDINEN**
METHOD FOR PRODUCING 4-PENTAFLUORIDE-SULFANYL-BENZOYLGUANIDINES
PROCEDE DE PRODUCTION DE 4-PENTAFLUOROSULFANYLBENZOYLGUANIDINES

(30) Priorität: 13.11.2003 DE 10353204
(43) Veröffentlichungstag der Anmeldung: 02.08.2006
(73) Patentinhaber: Sanofi-Aventis Deutschland GmbH, 65929 Frankfurt am Main (DE)
(72) Erfinder: SCHUBERT, Gerrit, 65779 Kelkheim (DE); KLEEMANN, Heinz-Werner, 65474 Bischofsheim (DE)
(86) Internationale Anmeldenummer: PCT/EP2004/012395
(87) Internationale Veröffentlichungsnummer: WO 2005/047241

(56) Entgegenhaltungen:
- EP-A- 0 589 336
- DE-A1- 10 222 192
- DE-A1- 19 711 953
- GB-A- 2 276 379
- M. GRAY, ET AL.: "Practical methylation of aryl halides by Suzuki-Miyaura coupling" TETRAHEDRON LETTERS, Bd. 41, Nr. 32, 5. August 2000 (2000-08-05), Seiten 6237-6240, XP004243549 ELSEVIER SCIENCE PUBLISHERS, AMSTERDAM, NL ISSN: 0040-4039

## Beschreibung

Die vorliegende Erfindung betrifft ein Verfahren zur Herstellung von 4-Pentafluorsulfanyl-benzoylguanidinen der Formel I. Die Verbindungen der Formel I sind NHE1-Inhibitoren und können, beispielsweise, zur Behandlung von Herz-Kreislauferkrankungen eingesetzt werden.

Die Patentanmeldung EP589336 offenbart ein Verfahren zur Herstellung von bestimmten Benzoylguanidin-Derivaten aus Benzoesäure-Derivaten, die keine Pentafluorosulfanyl-Gruppe enhalten. In der Anmeldung DE10222192 werden Pentafluorsulfanyl-benzoylguanidine als NHE1-Inhibitoren beschrieben. Die dort beschriebenen Verfahren zur Herstellung dieser Verbindungen verlaufen jedoch mit geringer Ausbeute und benötigen Reagenzien und Reaktionsbedingungen, die für eine Herstellung im größeren Maßstab einen großen technischen Aufwand erfordern oder ungeeignet sind.

Es wurde nun gefunden, dass sich die genannten Nachteile durch eine neue effiziente Synthese vermeiden lassen, die von käuflich erhältlichem 4-Nitrophenyl-schwefelpentafluorid ausgeht.

Die vorliegende Erfindung betrifft somit ein Verfahren zur Herstellung von Verbindungen der Formel I 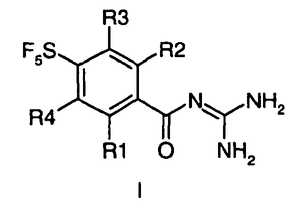 worin bedeuten:
- R1: Wasserstoff, Alkyl mit 1, 2, 3 oder 4 C-Atomen, Alkoxy mit 1, 2, 3 oder 4 C-Atomen, NR10R11, -Oₚ-(CH₂)ₙ-(CF₂)ₒ-CF₃ oder -(SOₘ)_{q}-(CH₂)ᵣ-(CF₂)ₛ-CF₃;
R10 und R11 unabhängig voneinander Wasserstoff, Alkyl mit 1, 2, 3 oder 4 C-Atomen oder -CH₂-CF₃;
m Null, 1 oder 2
n, o, p, q, r und s unabhängig voneinander Null oder 1;
- R2: Wasserstoff, -(SOₕ)_{z}-(CH₂)ₖ -(CF₂)ₗ -CF₃, Alkyl mit 1, 2, 3, 4, 5 oder 6 C-Atomen, Cycloalkyl mit 3, 4, 5, 6, 7 oder 8 C-Atomen, in dem 1, 2, 3 oder 4 Wasserstoff-Atome durch Fluoratome ersetzt sein können;
h Null, 1 oder 2;
z Null oder 1;
k Null, 1, 2, 3 oder 4;
l Null oder 1;
oder
- R2: -(CH₂)ₜ-Phenyl oder -O-Phenyl,
die unsubstituiert sind oder substituiert sind mit 1, 2 oder 3 Resten ausgewählt aus der Gruppe bestehend aus -Oᵤ-(CH₂)ᵥ-CF₃, Alkoxy mit 1, 2, 3 oder 4 C-Atomen, Alkyl mit 1,2,3 oder 4 C-Atomen und -SO₂CH₃;
t Null, 1, 2, 3 oder 4;
u Null oder 1;
v Null, 1, 2 oder 3;
oder
- R2: -(CH₂)_{w}-Heteroaryl,
das unsubstituiert ist oder substituiert ist mit 1, 2 oder 3 Resten ausgewählt aus der Gruppe bestehend aus -Oₓ-(CH₂)y-CF₃, Alkoxy mit 1, 2, 3 oder 4 C-Atomen und Alkyl mit 1, 2, 3 oder 4 C-Atomen, -SO₂CH₃;
w Null, 1, 2, 3 oder 4;
x Null oder 1;
y Null, 1, 2 oder 3;
- R3 und R4: unabhängig voneinander Wasserstoff oder F;
sowie deren Salze;
dadurch gekennzeichnet, dass, wie in Schema 1 dargestellt, 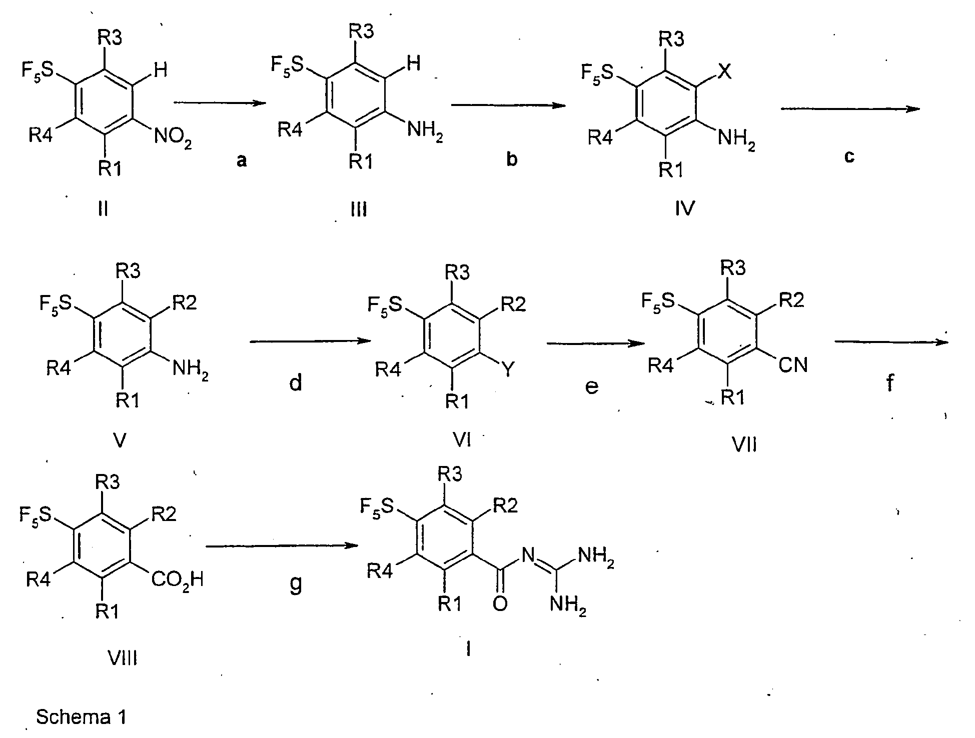
a) ein 4-Nitrophenyl-schwefelpentafluorid-Derivat der Formel II zum Amin der Formel III reduziert wird, und
b) die Verbindung der Formel III in ortho-Stellung zur Aminogruppe mit einem Halogenierungsmittel zur Verbindung der Formel IV halogeniert wird, und
c) in der Verbindung der Formel IV mit.einem geeigneten Nukleophil oder einer Organoelementverbindung, beispielsweise einer Alkylborverbindung, gegebenenfalls unter Katalyse, der Halogen-Substituent durch einen Substituenten R2 ersetzt wird, und
d) in der Verbindung der Formel V die Aminofunktion durch einen HalogenSubstituenten ersetzt wird, und
e) in der Verbindung der Formel VI der Halogen-Substituent durch eine Nitrilfunktion ersetzt wird, und
f) die Nitrilfunktion der Verbindung der Formel VII zur Carbonsäure hydrolysiert wird, und
g) die Carbonsäure der Formel VIII in das Acylguanidin der Formel I überführt wird, wobei in den Verbindungen der Formeln II, III, IV, V, VI, VII und VIII
R1 bis R4 definiert sind wie in Formel I und
X und Y unabhängig voneinander F, Cl, Br oder I sind.

In einer Ausführungsform sind Verbindungen der Formel I bevorzugt, in denen R1 durch Wasserstoff, Alkyl mit 1, 2, 3 oder 4 C-Atomen, Methoxy, Ethoxy, NR10R11, -O-CH₂-CF₃ oder -SOₘ-(CH₂)ᵣCF₃, wobei R10 und R11 unabhängig voneinander Wasserstoff, Alkyl mit 1, 2, 3 oder 4 C-Atomen oder -CH₂-CF₃ sind und wobei m Null, 1 oder 2 und r Null oder 1 ist, beschrieben wird, besonders bevorzugt sind Verbindungen, in denen R1 durch Wasserstoff oder Methyl beschrieben werden. In einer weiteren Ausführungsform sind Verbindungen der Formel I bevorzugt, in denen R2 durch Wasserstoff, Alkyl mit 1, 2, 3 oder 4 C-Atomen oder -SOₕ-(CH₂)ₖ-CF₃, wobei h Null, 1 oder 2 und k Null oder 1 ist, Phenyl oder -O-Phenyl, die unsubstituiert oder wie angegeben substituiert sind, beschrieben wird, besonders bevorzugt sind Verbindungen, in denen R2 durch Wasserstoff oder Methyl beschrieben wird.
In einer weiteren Ausführungsform sind Verbindungen der Formel I bevorzugt, in denen R3 und R4 durch Wasserstoff beschrieben werden.

Bei der Herstellung der Verbindungen der Formel I geht man so vor, dass zunächst in Schritt a (Schema 1) die Verbindungen der Formel II nach im Prinzip bekannten Methoden zur Reduktion von aromatischen Nitroverbindungen zu aromatischen Aminen zu Verbindungen der Formel III umgesetzt werden. Solche Methoden sind beispielsweise beschrieben in: R.C. Larock, Comprehensive Organic Transformations: A Guide to Functional Group Preparations, VCH Publishers, New York, Weinheim, 1999, 821-828 und der dort zitierten Literatur.

Anschließend (Schritt b) werden die Verbindungen der Formel III in einem organischen Lösungsmittel A gelöst und mit einem Halogenierungsmittel, beispielsweise mit einem Bromierungsmittel, umgesetzt. Die Reaktionstemperatur beträgt dabei im allgemeinen -30 °C bis +150 °C, bevorzugt 0 °C bis 40 °C. Die Reaktionszeit liegt im allgemeinen bei 10 min bis 20 h, je nach Zusammensetzung des Gemisches und des gewählten Temperaturbereiches. Zur Aufarbeitung kann das erhaltene Reaktionsgemisch anschließend über eine Kieselgelschicht filtriert, mit organischem Lösungsmittel A nachgewaschen und nach dem Entfernen des Lösungsmittels im Vakuum das Produkt durch übliche Reinigungsmethoden wie Umkristallisieren, Destillation oder Chromatographie gereinigt werden.
Es werden beispielsweise 0,1 bis 10 Mol der Verbindung der Formel II in 1000 mL organischem Lösungsmittel A gelöst. Beispielsweise verwendet man auf 1 Mol der zu halogenierenden Verbindung der Formel II 0,8 bis 1,2 Äquivalente des Halogenierungsmittels.
Unter dem Begriff "Halogenierungsmittel" werden zum Beispiel elementare Halogene, Halogen-Amin-Komplexe, cyclische und nichtcyclische N-halogenierte Carbonsäure-amide und -imide sowie Harnstoffe verstanden, wie sie beispielsweise in R.C. Larock, Comprehensive Organic Transformations: A Guide to Functional Group Preparations, VCH Publishers, New York, Weinheim, 1999, 619-628 und der dort zitierten Literatur oder M.B. Smith and J. March, March's Advanced Organic Chemistry: Reactions, Mechanisms, and Structure, Wiley, New York, 2001, 704-707 und der dort zitierten Literatur beschrieben sind, wie zum Beispiel N-Bromsuccinimid, N-Chlorsuccinimid, HBr in H₂SO₄ oder 1,3-Dibrom-5,5-dimethyl-imidazolidin-2,4-dion, wobei letzteres 2 Bromatome pro Molekül übertragen kann. Unter dem Begriff "Bromierungsmittel" werden zum Beispiel elementares Brom, Brom-Amin-Komplexe, cyclische und nichtcyclische N-bromierte Carbonsäure-amide und -imide sowie Harnstoffe verstanden, wie sie beispielsweise in R.C. Larock, Comprehensive Organic Transformations: A Guide to Functional Group Preparations, VCH Publishers, New York, Weinheim, 1999, 622-624 und der dort zitierten Literatur oder M.B. Smith and J. March, March's Advanced Organic Chemistry: Reactions, Mechanisms, and Structure, Wiley, New York, 2001, 704-707 und der dort zitierten Literatur beschrieben sind, beispielsweise N-Bromsuccinimid, HBr in H₂SO₄ oder 1,3-Dibrom-5,5-dimethylimidazolidin-2,4-dion, wobei letzteres 2 Bromatome pro Molekül übertragen kann. Unter dem Begriff "organisches Lösungsmittel A" werden vorzugsweise aprotische Lösungsmittel wie beispielsweise Dichlormethan, Chloroform, Tetrachlormethan, Pentan, Hexan, Heptan, Oktan, Benzol, Toluol, Xylol, Chlorbenzol, 1,2-Dichlorethan, Trichlorethylen oder Acetonitril verstanden.

Eventuell bei der Reaktion entstehendes HX kann durch organische oder anorganische Basen abgefangen werden.

In Schritt c werden die Verbindungen der Formel IV anschließend in einem organischen Lösungsmittel B gelöst und mit einen Nukleophil R2- oder einer Elementverbindung, die den Substituenten R2 enthält, zu Verbindungen der Formel V umgestzt. Dabei kann unter Zusatz einer Base A gearbeitet werden und ein katalysierendes Metallsalz A zugegeben werden.
Die Reaktionstemperatur beträgt dabei im allgemeinen zwischen -20°C und +150 °C, bevorzugt zwischen 30 °C und 100 °C. Die Reaktionszeit liegt im allgemeinen bei 0,5 h bis 20 h, je nach Zusammensetzung des Gemisches und des gewählten Temperaturbereiches. Zur Aufarbeitung kann das erhaltene Reaktionsgemisch anschließend über eine Kieselgelschicht abfiltriert, mit einem organischen Lösungsmittel B nachgewaschen und nach dem Entfernen des Lösungsmittels im Vakuum das Produkt durch übliche Reinigungsverfahren wie Umkristallisieren, Chromatographie, zum Beispiel an Kieselgel, Destillation oder Wasserdampfdestillation gereinigt werden.
Es werden beispielsweise 0,1 bis 10 Mol der Verbindung der Formel IV in 1000 mL organischem Lösungsmittel B gelöst. Beispielsweise verwendet man auf 1 Mol der Ausgangsverbindung der Formel IV 0,8 bis 3 Äquivalente'des Nucleophils R2- oder der Elementverbindung, die den Substituenten R2 enthält.
Unter dem Begriff "Nucleophil R2-" werden Verbindungen verstanden, die beim Deprotonieren einer Verbindung R2-H mit starken Basen, wie beispielsweise Alkyl- oder Aryllithiumverbindungen, Organomagnesiumverbindungen, Alkoholaten oder Lithiumdiisopropylamid, entstehen. Unter "Organoelementverbindungen, die den Substituenten R2 enthalten" werden beispielsweise Organolithiumverbindungen R2-Li, Organomagnesiumverbindungen R2-Mg-Hal, mit Hal = Cl, Br, I, Organoborverbindungen wie R2-B(OH)₂, R2-Boronsäureester wie beispielsweise

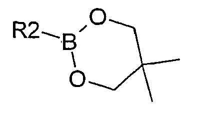

R2-Boronsäureanhydride wie beispielsweise 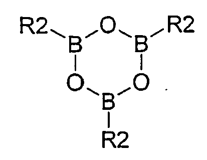 oder Organozinkverbindungen R2-Zn-Z, mit Z = Cl, Br, 1, verstanden.
Unter dem Begriff "Base A" werden Basen verstanden, wie sie als Hilfsbasen bei Kreuzkupplungsreaktionen verwendet werden und beispielsweise in A. Suzuki et al., Chem. Rev. 1995, 95, 2457-2483 oder M. Lamaire et al., Chem. Rev. 2002, 102, 1359-1469 oder S.P. Stanforth, Tetrahedron 1998, 54, 263-303 und der darin jeweils zitierten Literatur aufgeführt sind, beispielsweise Na₂CO₃, Cs₂CO₃, KOH, NaOH, K₃PO₄, N(Ethyl)₃.
Unter dem Begriff "organisches Lösungsmitel B" werden protische oder aprotische Lösungsmittel verstanden wie Diethylether, Dimethoxyethan, THF, Alkohole, Wasser oder deren Gemische. In einer Ausführungsform sind Gemische mit Wasser bevorzugt.
Unter dem Begriff "katalysierendes Metallsalz A" werden unter anderem Pd- und Ni-Katalysatoren verstanden, wie sie für Suzuki- und Negishi-Reaktionen verwendet werden und beispielsweise in A. Suzuki et al., Chem. Rev. 1995, 95, 2457-2483 oder M. Lamaire et al., Chem. Rev. 2002, 102, 1359-1469 oder S.P. Stanforth, Tetrahedron 1998, 54, 263 oder G.C. Fu et al., J. Am. Chem. Soc. 2001, 123, 10099 oder G.C. Fu et al., J. Am. Chem. Soc. 2002, 124, 13662 und der dort jeweils zitierten Literatur beschrieben sind, einschließlich der zugesetzten Liganden, wie Pd(OAc)₂, PdCl₂(dppf) oder Pd₂(dba)₃.

In Schritt d werden anschließend die Verbindungen der Formel V durch ein Diazotierungs-Halogenierungsverfahren mit einem Diazotierungs-Halogenierungsmittel, beispielsweise mit einem Diazotierungs-Bromierungsmittel, wie es für andere aromatische Amine zum Austausch der Amin- gegen eine Halogen-Funktion beispielsweise in M.B. Smith and J. March, March's Advanced Organic Chemistry: Reactions, Mechanisms, and Structure, Wiley, New York, 2001, 935-936 oder R.C. Larock, Comprehensive Organic Transformations: A Guide to Functional Group Preparations, VCH Publishers, New York, Weinheim, 1999, 678-679 und der dort zitierten Literatur beschrieben wurde, beispielsweise durch die Sandmeyer- oder Gattermann-Reaktion, in die Verbindungen der Formel VI überführt. Bevorzugt ist das Verfahren von M. Doyle et al., J. Org. Chem. 1977, 42, 2426 oder von S. Oae et al., Bull. Chem. Soc. Jpn. 1980, 53, 1065.

In Schritt e werden die Verbindungen der Formel VI in einem Löungsmittel C mit einem Cyanierungsmittel, zum Beispiel unter Zusatz eines katalysierenden Metallsalzes B, umgesetzt. Die Reaktionstemperatur beträgt im allgemeinen 20 °C bis 200 °C, bevorzugt 80 °C bis 150 °C. Die Reaktionszeit liegt im allgemeinen bei 1 h bis 20 h, je nach Zusammensetzung des Gemisches und des gewählten Temperaturbereiches. Die erhaltenen Reaktionsgemische können über eine Kieselgel- oder Kieselgurschicht abgesaugt und das Filtrat wässrig-extraktiv aufgearbeitet. Nach dem Eindampfen des Lösungsmittels im Vakuum wird die Verbindung der Formel VII durch übliche Reinigungsverfahren wie Umkristallisieren, Chromatographie an Kieselgel, Destillation oder Wasserdampfdestillation gereinigt.
Es werden beispielsweise 0,1 bis 10 Mol der Verbindung der Formel VI in 1000 mL organischem Lösungsmittel C gelöst. Beispielsweise verwendet man auf 1 Mol der umzusetzenden Verbindungen mit der Formel VI 1 bis 10 Äquivalente des Cyanierungsmittels.
Unter dem Begriff "Cyanierungsmittel" werden beispielsweise Alkalimetallcyanide oder Zn(CN)₂ entweder alleine oder im Gemisch mit metallischem Zink, bevorzugt in Form von Zink-Staub, verstanden.
Unter dem Begriff "organisches Lösungsmittel C" werden vorzugsweise aprotische polare Lösungsmittel wie beispielsweise DMF, Dimethylacetamid, NMP, DMSO verstanden.
Unter dem Begriff "katalysierendes Metallsalz B" werden unter anderem Pd- und Ni-Katalysatoren verstanden, wie sie bei Suzuki-Reaktionen eingesetzt werden und beispielsweise in A. Suzuki et al., Chem. Rev. 1995, 95, 2457-2483 oder M. Lamaire et al., Chem. Rev. 2002, 102, 1359-1469 oder S.P. Stanforth, Tetrahedron 1998, 54, 263 und der dort zitieten Literatur beschrieben werden, beispielsweise PdCl₂(dppf), Pd(OAc)₂, Pd₂(dba)₃.

Anschließend werden die erhaltenen Verbindungen der Formel VII in Schritt f, zum Beispiel in Gegenwart einer Base, zu den Carbonsäuren der Formel VIII hydrolysiert. Dies kann geschehen durch dem Fachmann bekannte Verfahren zur Hydrolyse aromatischer Nitrile, wie sie beispielsweise in R.C. Larock, Comprehensive Organic Transformations: A Guide to Functional Group Preparations, VCH Publishers, New York, Weinheim, 1999, 1986-1987 oder M.B. Smith and J. March, March's Advanced Organic Chemistry: Reactions, Mechanisms, and Structure, Wiley, New York, 2001, 1179-1180 und der dort zitierten Literatur beschrieben sind.

In Schritt g werden die Carbonsäuren der Formel VIII dann anschließend in die Acylguanidine mit der Formel IX überführt. Dazu werden die Carbonsäuren in aktivierte Säurederivate überführt, wie Carbonsäurehalogenide, vorzugsweise Carbonsäurechloride, Ester, vorzugsweise Methylester, Phenylester, Phenylthioester, Methylthioester, 2-Pyridylthioester, oder einen Stickstoffheterocyclus, vorzugsweise 1-lmidazolyl. Die Ester und Stickstoffheterocyclen erhält man vorteilhaft in dem Fachmann bekannter Weise aus den zugrundeliegenden Carbonsäurechloriden, die man ihrerseits wiederum in bekannter Weise aus den zugrundeliegenden Carbonsäuren, beispielsweise mit Thionylchlorid, herstellen kann.
Neben den Carbonsäurechloriden lassen sich auch weitere aktivierte Säurederivate in bekannter Weise direkt aus den zugrundeliegenden Benzoesäuren herstellen, wie die Methylester durch Behandeln mit gasförmigem HCl in Methanol, die Imidazolide durch Behandeln mit Carbonyldiimidazol die gemischten Anhydride mit Cl-COOC₂H₅ oder Tosylchlorid in Gegenwart von Triethylamin in einem inerten Lösungsmittel, wie auch Aktivierungen von Benzoesäuren mit Dicyclohexylcarbodiimid (DCC) oder mit O-[(Cyano(ethoxycarbonyl)methylen)amino]-1,1,3,3-tetramethyluronium-tetrafluorborat ("TOTU") möglich sind. Eine Reihe geeigneter Methoden zur Herstellung von aktivierten Carbonsäurederivaten sind unter Angabe von Quellenliteratur M.B. Smith and J. March, March's Advanced Organic Chemistry: Reactions, Mechanisms, and Structure, Wiley, New York, 2001, 506-516 oder R.C. Larock, Comprehensive Organic Transformations: a Guide to Functional Group Preparations, VCH Publishers, New York, Weinheim, 1999, 1941-1949 angegeben.

Die Umsetzung eines aktivierten Carbonsäurederivates mit Guanidin erfolgt vorzugsweise in an sich bekannter Weise in einem protischen oder aprotischen polaren aber inerten organischen Lösungsmittel entweder mit freier Guanidin-Base oder mit Guanidinium-Chlorid in Gegenwart einer Base. Dabei haben sich bei der Umsetzung der Benzoesäuremethylester mit Guanidin Methanol, Isopropanol oder THF bei Temperaturen von 20°C bis zur Siedetemperatur dieser Lösungsmittel bewährt. Bei den meisten Umsetzungen von Carbonsäurederivaten mit salzfreiem Guanidin wird vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung mit Guanidin verwendet werden.
Wenn als Carbonsäurederivat ein Carbonsäurechlorid eingesetzt wird, arbeitet man vorteilhaft unter Zusatz eines Säurefängers, zum Beispiel in Form von überschüssigem Guanidin, zur Abbindung der Halogenwasserstoffsäure.

Für die Darstellung der Verbindungen der Formel 1, in denen R2 Wasserstoff ist, können erfolgt die Synthese ohne die Schritte b und c.
Um Verbindungen der Formel I mit R2 = -(SOₕ)_{z}-(CH₂)ₖ -(CF₂)ₗ -CF₃, wobei h 1 oder 2 ist, herzustellen werden wie oben dargestellt Verbindungen synthetisiert, in denen R2 -(SOₕ)_{z}-(CH₂)ₖ -(CF₂)ₗ -CF₃ ist, wobei h Null ist, und anschließend durch allgemein bekannte Oxidationsreaktionen in die gewünschten Verbindungen der Fomel I überführt werden.

Die Aufarbeitung des Reaktionsgemisches kann nach jedem der Verfahrensschritte a), b), c), d), e), f) und g) erfolgen oder nach zwei oder mehren Verfahrensschritten. Die Synthese der Verbindungen der Formel I nach dem erfindungsgemäßen Verfahren kann aber auch in zwei oder mehreren aufeinanderfolgenden Verfahrensschritten ohne Isolierung der in den einzelnen Verfahrensschritte erhaltenen Verbindungen III, IV, V, VI, VII oder VIII erfolgen, wobei eine Aufarbeitung nicht nach jedem Verfahrensschritt durchgeführt werden muss. Die Aufarbeitung und gewünschtenfalls die Reinigung der Produkte erfolgt nach den üblichen Methoden wie Extraktion, pH-Trennung, Chromatographie oder Kristallisation und den üblichen Trocknungen.
Die Ausgangsverbindungen der Formeln II sind käuflich erhältlich oder können nach oder analog zu in der Literatur beschriebenen, dem Fachmann bekannten Verfahren hergestellt werden, zum Beispiel wie beschrieben in Bowden, R.D., Comina, P.J., Greenhall, M.P., Kariuki, B.M., Loveday, A., Philip, D. Tetrahedron 2000, 56, 5660. In den Ausgangsverbindungen können auch funktionelle Gruppen in geschützter Form oder in Form von Vorstufen vorliegen und dann in den nach dem erfindungsgemäßen Verfahren hergestellten Verbindungen der Formel I in die gewünschten Gruppen überführt werden. Entsprechende Schutzgruppentechniken sind dem Fachmann bekannt. Beispielsweise kann in Verbindungen der Formel II, in denen R1 für NH₂ steht, die Gruppe NH₂ in durch eine Acetyl-, Trifluoracetyl- oder Tritylgruppe geschützter Form vorliegen und wieder entschützt werden.

Die Schritte a und b des in Schema 1 dargestellten Verfahrens sind bereits aus GB2276379 bekannt. Einige weitere Teilschritte der Synthese gemäß Schema 1 wurden bereits in ähnlicher Weise für andere Verbindungen in M.Gray et al., Tetrahedron Letters, 2000, 41 (32), 6237-6240 und DE19711953 beschrieben.

Ein weiterer Aspekt der Erfindung betrifft neue Verbindungen der Formeln V, VI, VII und VIII.

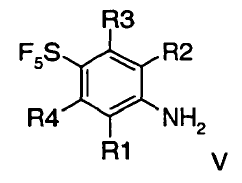

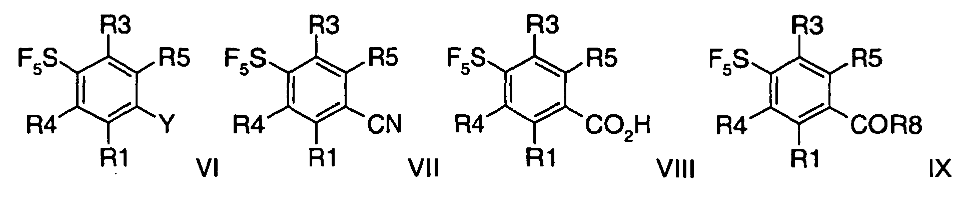

Die Erfindung betrifft somit 4-Pentafluorsulfanyl-substituierte Verbindungen der Formel X 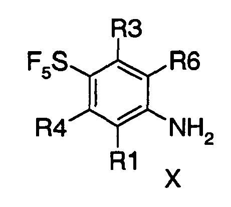 worin bedeuten
- R1: Wasserstoff, Alkyl mit 1, 2, 3 oder 4 C-Atomen, Alkoxy mit 1, 2, 3 oder 4 C-Atomen, NR10R11, -Op-(CH₂)ₙ-(CF₂)ₒ-CF₃ oder -(SOₘ)_{q}-(CH₂)ᵣ-(CF₂)ₛ-CF₃;
R10 und R11 unabhängig voneinander Wasserstoff, Alkyl mit 1, 2, 3 oder 4 C-Atomen oder -CH₂-CF₃;
m Null, 1 oder 2
n, o, p, q, r und s unabhängig voneinander Null oder 1;
- R6: -(SOₕ)_{z}-(CH₂)ₖ -(C₂)ₗ -CF₃, Alkyl mit 1, 2, 3, 4, 5 oder 6 C-Atomen, Cycloalkyl mit 3, 4, 5, 6, 7 oder 8 C-Atomen, in dem 1, 2, 3 oder 4 Wasserstoff-Atome durch Fluoratome ersetzt sein können;
h Null, 1 oder 2;
z Null oder 1;
k Null, 1, 2, 3 oder 4;
l Null oder 1;
oder
- R6: -(CH₂)ₜ-Phenyl oder -O-Phenyl,
die unsubstituiert sind oder substituiert sind mit 1, 2 oder 3 Resten ausgewählt aus der Gruppe bestehend aus -Oᵤ-(CH₂)ᵥ-CF₃, Alkoxy mit 1, 2, 3 oder 4 C-Atomen, Alkyl mit 1, 2, 3 oder 4 C-Atomen und -SO₂CH₃;
t Null, 1, 2, 3 oder 4;
u Null oder 1;
v Null, 1, 2 oder 3;
oder
- R6: -(CH₂)_{w}-Heteroaryl,
das unsubstituiert ist oder substituiert ist mit 1, 2 oder 3 Resten ausgewählt aus der Gruppe bestehend aus -Oₓ-(CH₂)_{y}-CF₃, Alkoxy mit 1, 2, 3 oder 4 C-Atomen und Alkyl mit 1,2,3 oder 4 C-Atomen, -SO₂CH₃;
w Null, 1, 2, 3 oder 4;
x Null oder 1;
y Null, 1, 2 oder 3;
- R3 und R4: unabhängig voneinander Wasserstoff oder F;
sowie deren Salze;

In einer Ausführungsform sind Verbindungen der Formel X bevorzugt, in denen R1 durch Wasserstoff, Alkyl mit 1, 2, 3 oder 4 C-Atomen, Methoxy, Ethoxy, NR10R11, -O-CH₂-CF₃ oder -SOₘ-(CH₂)ᵣ-CF₃, wobei R10 und R11 unabhängig voneinander Wasserstoff, Alkyl mit 1, 2, 3 oder 4 C-Atomen oder -CH₂-CF₃ sind und wobei m Null, 1 oder 2 und r Null oder 1 ist, beschrieben wird, besonders bevorzugt sind Verbindungen, in denen R1 durch Wasserstoff oder Methyl beschrieben werden. In einer weiteren Ausführungsform sind Verbindungen der Formel X bevorzugt, in denen R6 durch Wasserstoff, F, Cl, Br, I, Alkyl mit 1, 2, 3 oder 4 C-Atomen oder-SOₕ-(CH₂)ₖ-CF₃, wobei h Null, 1 oder 2 und k Null oder 1 ist, Phenyl oder -O-Phenyl, die unsubstituiert oder wie angegeben substituiert sind, beschrieben wird, besonders bevorzugt sind Verbindungen, in denen R6 durch Wasserstoff oder Methyl beschrieben wird, beispielsweise durch Wasserstoff. In einer weiteren Ausführungsform sind Verbindungen der Formel X bevorzugt, in denen R6 durch F, Cl, Br oder I beschrieben wird, insbesondere durch Br.
In einer weiteren Ausführungsform sind Verbindungen der Formel X bevorzugt, in denen R3 und R4 durch Wasserstoff beschrieben werden.

Die Erfindung betrifft ebenfalls 4-Pentafluorsulfanyl-substituierte Verbindungen der Formel XI 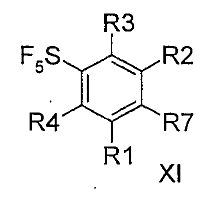 worin bedeuten
- R1: Wasserstoff, Alkyl mit 1, 2, 3 oder 4 C-Atomen, Alkoxy mit 1, 2, 3 oder 4 C-Atomen, NR10R11, -Oₚ-(CH₂)ₙ-(CF₂)ₒ-CF₃ oder -(SOₘ)_{q}-(CH₂)ᵣ-(CF₂)ₛ-CF₃;
R10 und R11 unabhängig voneinander Wasserstoff, Alkyl mit 1, 2, 3 oder 4 C-Atomen oder -CH₂-CF₃;
m Null, 1 oder 2
n, o, p, q, r und s unabhängig voneinander Null oder 1;
- R2: Wasserstoff, F, Cl, Br, I, -(SOₕ)_{z}-(CH₂)ₖ -(CF₂)ₗ -CF₃ oder Alkyl mit 1, 2, 3, 4, 5 oder 6 C-Atomen;
h Null, 1 oder 2;
z Null oder 1;
k Null, 1, 2, 3 oder 4;
l Null oder 1;
- R3 und R4: unabhängig voneinander Wasserstoff oder F;
- R7: CN;
sowie deren Salze.

In einer Ausführungsform sind Verbindungen der Formel XI bevorzugt, in denen R1 durch Wasserstoff, Alkyl mit 1, 2, 3 oder 4 C-Atomen, Methoxy, Ethoxy, NR10R11, -O-CH₂-CF₃ oder-SOₘ-(CH₂)ᵣ-CF₃, wobei R10 und R11unabhängig voneinander Wasserstoff, Alkyl mit 1, 2, 3 oder 4 C-Atomen oder -CH₂-CF₃ sind und wobei m Null, 1 oder 2 und r Null oder 1 ist, beschrieben wird, besonders bevorzugt sind Verbindungen, in denen R1 durch Wasserstoff oder Methyl beschrieben werden. In einer weiteren Ausführungsform sind Verbindungen der Formeln XI bevorzugt, in denen R2 durch Wasserstoff, F, Cl, Br, 1, Alkyl mit 1, 2, 3 oder 4 C-Atomen oder -SOₕ-(CH₂)ₖ-CF₃, wobei h Null, 1 oder 2 und k Null oder 1 ist, beschrieben wird, besonders bevorzugt sind Verbindungen, in denen R2 durch Wasserstoff oder Methyl beschrieben wird, beispielsweise durch Wasserstoff. In einer weitern Ausführungsform sind Verbindungen der Formel XI bevorzugt, in denen R2 durch F, Cl, Br oder I beschrieben wird, insbesondere durch Br.
In einer weiteren Ausführungsform sind Verbindungen der Formel XI bevorzugt, in denen R3 und R4 durch Wasserstoff beschrieben werden.

Enthalten die Substituenten R1, R2, R3, R4 und R6 ein oder mehrere Asymmetriezentren, so können diese unabhängig voneinander sowohl S als auch R konfiguriert sein. Die Verbindungen können als optische Isomere, als Diastereomere, als Racemate oder als Gemische derselben in allen Verhältnissen vorliegen.

Die vorliegende Erfindung umfasst alle tautomeren Formen der Verbindungen der Formel I.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl), tert-Butyl (= 1,1-Dimethylethyl), n-Pentyl, Isopentyl, tert-Pentyl, Neopentyl und Hexyl. Bevorzugte Alkylreste sind Methyl, Ethyl, n-Propyl und Isopropyl. Substituierte Alkylreste können in beliebigen Positionen substituiert sein.

Beispiele für Cycloalkylreste sind Cyclopropyl, Cyclobutyl, Cyclopentyl, Cyclohexyl Cycloheptyl oder Cyclooctyl. Substituierte Cycloalkylreste können in beliebigen Positionen substituiert sein.

Phenylreste können unsubstituiert sein oder einfach oder mehrfach, zum Beispiel einfach, zweifach oder dreifach, durch gleiche oder verschiedene Reste substituiert sein. Wenn ein Phenylrest substituiert ist, trägt er bevorzugt einen oder zwei gleiche oder verschiedene Substituenten. Dies gilt ebenso für substituierte Phenylreste in Gruppen wie zum Beispiel Phenylalkyl oder Phenyloxy. In monosubstituierten Phenylresten kann sich der Substituent in der 2-Position, der 3-Position oder der 4-Position befinden. Zweifach substituiertes Phenyl kann in 2,3-Position, 2,4-Position, 2,5-Position, 2,6-Position, 3,4-Position oder 3,5-Position substituiert sein. In dreifach substituierten Phenylresten können sich die Substituenten in 2,3,4-Position, 2,3,5-Position, 2,4,5-Position, 2,4,6-Position, 2,3,6-Position oder 3,4,5-Position befinden. Heteroarylreste sind aromatische Ringverbindungen, in denen ein oder mehrere Ringatome Sauerstoffatome, Schwefelatome oder Stickstoffatome sind, z. B. 1, 2 oder 3 Stickstoffatome, 1 oder 2 Sauerstoffatome, 1 oder 2 Schwefelatome oder eine Kombinationen aus verschiedenen Heteroatomen. Die Heteroarylreste können über alle Positionen angebunden sein, zum Beispiel über 1-Position, 2-Position, 3-Position, 4-Position, 5-Position, 6-Position, 7-Position oder 8-Position. Heteroarylreste können unsubstituiert sein oder einfach oder mehrfach, zum Beispiel einfach, zweifach oder dreifach, durch gleiche oder verschiedene Reste substituiert sein. Dies gilt ebenso für die Heteroarylreste wie zum Beispiel im Rest Heteroarylalkyl. Heteroaryl bedeutet zum Beispiel Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl und Cinnolinyl.

Als Heteroarylreste gelten insbesondere 2- oder 3-Thienyl, 2- oder 3-Furyl, 1-, 2- oder 3- Pyrrolyl, 1-, 2-, 4- oder 5-Imidazolyl, 1-, 3-, 4- oder 5-Pyrazolyl, 1,2,3-Triazol-1-, -4- oder -5-yl, 1,2,4-Triazol-1-, -3- oder -5-yl, 1- oder 5-Tetrazolyl, 2-, 4- oder 5-Oxazolyl, 3-, 4- oder 5-Isoxazolyl, 1,2,3-Oxadiazol-4- oder -5-yl, 1,2,4-Oxadiazol-3- oder -5-yl, 1,3,4-Oxadiazol-2-yl oder -5-yl, 2-, 4- oder 5-Thiazolyl, 3-, 4- oder 5-Isothiazolyl, 1,3,4-Thiadiazol-2- oder -5-yl, 1,2,4-Thiadiazol-3- oder -5-yl, 1,2,3-Thiadiazol-4- oder -5-yl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 1-, 2-, 4- oder 5-Benzimidazolyl, 1-, 3-, 4-, 5-, 6- oder 7-Indazolyl, 2-, 3-, 4-, 5-, 6-, 7- oder 8-Chinolyl, 1-, 3-, 4-, 5-, 6-, 7- oder 8-Isochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl. Umfasst sind weiterhin die entsprechenden N-Oxide dieser Verbindungen, also zum Beispiel 1-Oxy-2-, 3- oder 4-pyridyl.

Besonders bevorzugt sind die Heteroaromaten 2- oder 3-Thienyl, 2- oder 3-Furyl, 1-, 2- oder 3- Pyrrolyl, 1-, 2-, 4- oder 5-Imidazolyl, 2-, 3-, 4-, 5-, 6-, 7- oder 8-Chinolyl, 1-, 3-, 4- oder 5-Pyrazolyl, 2-, 3- oder 4-Pyridyl, 2- oder 3-Pyrazinyl, 2-, 4-, 5- oder 6-Pyrimidinyl und 3- oder 4-Pyridazinyl.

Die Verbindungen der Formel I können in Form ihrer Salze isoliert werden. Diese werden nach den üblichen Methoden durch Umsetzung mit Säuren oder Basen erhalten. Als Säureadditionssalze kommen dabei beispielsweise Halogenide, insbesondere Hydrochloride, Hydrobromide, Lactate, Sulfate, Citrate, Tartrate, Acetate, Phosphate, Methylsulfonate, Benzolsulfonate, p-Toluolsulfonate, Adipinate, Fumarate, Gluconate, Glutamate, Glycerolphosphate, Maleinate, Benzoate, Oxalate und Pamoate und Trifluoracetate in Frage, im Fall der Herstellung von Wirkstoffen bevorzugt pharmazeutisch verträgliche Salze. Enthalten die Verbindungen eine Säuregruppe, können sie Salze mit Basen bilden, beispielsweise Alkalimetallsalze, vorzugsweise Natrium- oder Kaliumsalze, oder Ammoniumsalze, zum Beispiel als Salze mit Ammoniak oder organischen Aminen oder Aminosäuren. Sie können auch als Zwitterion vorliegen.

### Liste der Abkürzungen:

- DMF: N,N-Dimethylformamid
- DMSO: Dimethylsulfoxid
- dba: Dibenzylideneaceton
- OAc: Acetat
- Fp.: Schmelzpunkt
- MTB: tert.-Butyl-methylether
- NMP: N-Methyl-2-Pyrrolidon
- dppf: 1,1'-Bis-(diphenylphosphino)-ferrocen
- THF: Tetrahydrofuran

### Experimenteller Teil

### Beispiel 1:

### a) 4-Aminophenyl-schwefelpentafluorid

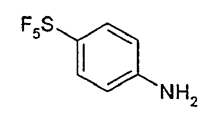

Eine Lösung von Zinn(II)chlorid (1465 g, 7,73 Mol) in konzentrierter (32-prozentiger) wässriger HCl-Lösung wurde unter Rühren auf 80 °C erwärmt und dann unter Eiskühlung innerhalb von 1 h in 8 Portionen 4-Nitrophenyl-schwefelpentafluorid (584 g, 2,344 Mol) eingetragen. Die Innentemperatur wurde hierbei unter 100 °C gehalten. Anschließend wurde das Gemisch 1,5 h bei 85 °C Innentemperatur gerührt und dann innerhalb einer weiteren Stunde auf 45 °C abgekühlt. Ein Gemisch aus Eis (12 kg), NaOH (2 kg) und Dichlormethan (1,5 L) wurde vorbereitet und das Reaktionsgemisch unter starkem Rühren zugegeben. Die Phasen wurden getrennt, die wässrige Phase wurde 3 mal mit je 1 L Dichlormethan extrahiert und die vereinigten organischen Phasen wurden über Na₂SO₄ getrocknet und im Vakuum eingedampft. Man erhielt 510 g (99 %) 4-Aminophenyl-schwefelpentafluorid als hellgelbes, kristallines Pulver, Fp. 63-65 °C (Bowden, R.D., Comina, P.J., Greenhall, M.P., Kariuki, B.M., Loveday, A., Philip, D. Tetrahedron 2000, 56, 3399: 57-59 °C).
¹H-NMR 400 MHz, CDCl₃: δ= 3.99 (bs, 2H), 6.61 (d, J=9 Hz, 2H), 7.52 (d, J=9 Hz, 2H) ppm.

### b) 4-Amino-3-brom-phenyl-schwefelpentafluorid

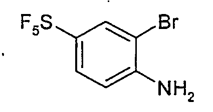

4-Aminophenyl-schwefelpentafluorid (510 g, 2,327 Mol) wurde in Dichlormethan (7 L) gelöst, die Lösung auf 5 °C abgekühlt und unter Rühren 1,3-Dibromo-5,5-dimethylimidazolidin-2,4-dion (326 g, 1,14 Mol) in mehreren Portionen unter Eiskühlung so eingetragen, dass die Innentemperatur bei 3-8 °C gehalten wurde (etwa 1 h). Anschließend wurde das Gemisch ohne äußere Kühlung 1 h rühren und auf Raumtemperatur aufwärmen gelassen. Das Gemisch wurde über ein Kieselgelbett (Volumen etwa 1 L) filtriert, mit Dichlormethan (5,5 L) nachgewaschen und das Filtrat im Vakuum eingedampft. Man erhielt etwa 700 g einer rotbraunen, kristallinen Masse, die bei 60 °C in n-Heptan (600 mL) gelöst wurde und danach im Kühlschrank bei 4 °C kristallisierte. Nach dem Absaugen erhielt man 590 g (85 %) 4-Amino-3-brom-phenylschwefelpentafluorid als bräunliche Kristalle,
Fp. 59-59,5 °C.
¹H-NMR 400 MHz, CDCl₃: δ = 4.45 (bs, 2H), 6.72 (d, J=9 Hz, 1H), 7.49 (dd, J₁=9 Hz, J₂=2.5 Hz, 1 H), 7.81 (d, J=2.5 Hz, 1 H) ppm.
C₆H₅BrF₅NS (298.07): ber. C 24.18, H 1.69, N 4.70; gef. C 24.39, H, 1.45, N 4.77.

### c) 4-Amino-3-methyl-phenyl-schwefelpentafluorid

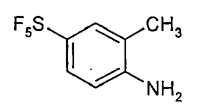

Ein Gemisch aus Cs₂CO₃ (794 g, 2,7 Mol), Dimethoxyethan (2 L), Wasser (300 mL) und Trimethylboroxin (50-prozentige Lösung in THF, 225 g, 0,9 Mol) wurde auf 70 °C erwärmt, PdCl₂(dppf) x CH₂Cl₂ (37 g, 45 mmol) zugegeben und eine Lösung von 4-Amino-3-brom-phenyl-schwefelpentafluorid (270 g, 0,9 Mol) in Dimethoxyethan (400 mL) innerhalb von 2 h unter Erhitzen des Reaktionsgemisches am Rückfluß zugetropft. Anschließend wurde weitere 3 h unter Rückfluß erhitzt, dann auf Raumtemperatur abgekühlt, mit MTB-Ether (500 mL) verdünnt, über eine Kieselgelsäule (14 x 7 cm, 70-200 µm) filtriert und mit MTB-Ether (2500 mL) nachgewaschen. Das Filtrat wurde im Vakuum eingedampft. Man erhielt 490 g einer schwarzen, halbkristallinen Masse, die einer Wasserdampfdestillation unterzogen wurde. Insgesamt wurden 5,5 L Kondensat gesammelt, aus dem das Produkt sich bereits kristallin abschied. Das Kondensat wurde 3 x mit MTB-Ether extrahiert, die vereinigten organischen Phasen über Na₂SO₄ getrocknet und im Vakuum eingedampft. Man erhielt 4-Amino-3-methyl-phenylschwefelpentafluorid (181 g, 76 %) als farblose Kristalle, Fp. 65-66 °C,
¹H-NMR 400 MHz, CDCl₃: δ = 2.18 (s, 3 H), 3.92 (bs, 2 H), 6.60 (d, J=8.5 Hz, 1 H), 7.40 (dd, J₁=8.5 Hz, J₂=2.5 Hz, 1 H), 7.43 (d, J=2.5 Hz, 1 H) ppm.
C₇H₈F₅NS (233,20): ber. C 36.05, H 3.46, N 6.01; gef. C 36.43 H 3.30 N 6.09.

### d) 4-Brom-3-methyl-phenyl-schwefelpentafluorid

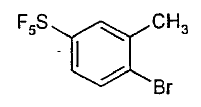

Ein Gemisch aus tert.-Butylnitrit (90-prozentig, 37 ml, 280 mmol) und CuBr₂ (35.8 g, 160 mmol) in Acetonitril (260 mL) wurde bei 5 °C vorgelegt und unter Rühren und Eiskühlung eine Lösung von 4-Amino-3-methyl-phenyl-schwefelpentafluorid (30,9 g, 132,5 mmol) in MTB-Ether (140 mL) innerhalb.1 h bei 5-8 °C zugetropft. Dabei setzte nach etwa 2 min Stickstoff-Entwicklung ein. Anschließend wurde das Gemisch innerhalb 1 h unter Rühren auf Raumtemperatur erwärmen gelassen, ein Gemisch aus Eis (250 g), 26-prozentiger wässriger NH₃-Lösung (50 mL) und MTB-Ether (250 mL) zugesetzt und das Gemisch 10 min gerührt. Die Phasen wurden getrennt, die wässrigen 3 x mit MTB-Ether (je 150 mL) extrahiert und die vereinigten organischen Phasen einmal mit 400 mL Wasser geschüttelt. Nach Trocknen mit Na₂SO₄ und Eindampfen der organischen Phase erhielt man 39 g 4-Brom-3-methyl-phenylschwefelpentafluorid als rotbraunes Öl, das laut 1 H-NMR mit 8 Mol-% 4,5-Dibrom-3-methyl-phenyl-schwefelpentafluorid verunreinigt war, aber ohne weitere Reinigung weiterverwendet wurde. Ausbeute 89 %, bezogen auf eine Reinheit von 90 %. Zur Verbrennungsanalyse wurde eine Probe durch Chromatographie an Kieselgel (35-70 µm, Heptan) gereinigt.
¹H-NMR 400 MHz, CDCl₃: δ = 2.47 (s, 3 H), 7.43 (dd, J₁=9 Hz, J₂=3 Hz, 1 H), 7.62 (m, 2 H) ppm. Signale von 4,5-Dibrom-3-methyl-phenyl-schwefelpentafluorid (Verunreinigung):
2.56 (s, 3 H), 7.56 (d, J=2.5 Hz, 1 H), 7.85 (d, J=2.5 Hz, 1 H).
C₇H₆BrF₅S (297.09): ber. C 28.30, H 2.04; gef. C 28.42, H 1.78.

### e) 4-Cyan-3-methyl-phenyl-schwefelpentafluorid

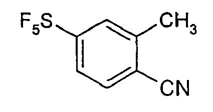

Ein Gemisch aus 4-Brom-3-methyl-phenyl-schwefelpentafluorid (136,4 g, Reinheit 80 %, 0,367 Mol), Zn(CN)₂ (72,8 g, 0,62 Mol) und Zn-Staub (7,2 g, 0,11 Mol) wurde in Dimethylacetamid (900 mL) und Wasser (40 mL) unter Stickstoff-Begasung vorgelegt, unter Rühren auf 125 °C erwärmt und PdCl₂(dppf) x CH₂Cl₂ (32,7 g, 40 mmol) zugesetzt. Nach einstündigem Rühren bei 125 °C wurde nochmals PdCl₂(dppf) x CH₂Cl₂ (16,3 g, 20 mmol) und Zn-Staub (3,6 g, 55 mmol) zugegeben und weitere 2 h bei 125 °C gerührt. Anschließend wurde auf Raumtemperatur abgekühlt, mit n-Heptan (400 mL) verdünnt und das Gemisch unter Zugabe von 5 N wässriger NH₄Cl-Lösung (250 mL) und Wasser (450 mL) 15 min lang stark gerührt. Das Gemisch wurde über eine Kieselgurschicht abgesaugt, die Phasen getrennt und die wässrige 2 x mit n-Heptan (200 mL) extrahiert. Die vereinigten organischen Phasen wurden mit Wasser (450 mL) geschüttelt, über MgSO₄ getrocknet und im Vakuum eingedampft. Der erhaltene schwarze Rückstand wurde in 200 mL n-Heptan gelöst, filtriert und erneut im Vakuum eingedampft. Man erhielt 78 g einer dunkelbraunen Flüssigkeit, die durch Chromatographie an einer Kieselgelsäule (7 x 55 cm, 60-200 µm, n-Heptan/Dichlormethan 4:1 bis 3:2) gereinigt wurde. Als erste Fraktion erhielt man 6,5 g 4-Brom-3-methyl-phenyl-schwefelpentafluorid (Edukt) als gelbliche Flüssigkeit und anschließend 71,1 g (80 %) 4-Cyan-3-methyl-phenyl-schwefelpentafluorid als hellgelbes Öl.
¹H-NMR 400 MHz, CDCl₃: δ = 2.65 (s, 3 H), 7.71 (m, 3 H) ppm.

### f) 2-Methyl-4-pentafluorsulfanyl-benzoesäure

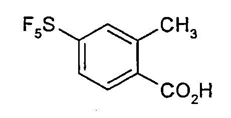

Ein Gemisch aus 4-Cyan-3-methyl-phenyl-schwefelpentafluorid (41,2 g, 169,4 g), NaOH (20,4 g, 510 mmol) und Wasser (60 mL) in Ethylenglykol (160 mL) wurde auf 130 °C erwärmt und 4 h bei dieser Temperatur gerührt. Anschließend wurde auf Raumtemperatur abgekühlt, mit MTB-Ether (150 mL) und Wasser (250 mL) verdünnt und das Gemisch abgesaugt. Die Phasen des Filtrats wurden getrennt und die wässrige mit konzentrierter wässriger HCl-Lösung angesäuert und der ausfallende Feststoff abgesaugt. Man erhielt 41,1 g (93 %) 2-Methyl-4-pentafluorsulfanylbenzoesäure als farblose Kristalle, Fp. 138-139 °C.
¹H-NMR 400 MHz, DMSO-d₆: δ = 2.60 (s, 3 H), 7.81 (dd, J₁=8.5 Hz, J₂=2 Hz, 1 H), 7.89 (d, J=2 Hz, 1 H), 7.97 (d, J=8.5 Hz, 1 H), 13.43 (bs, 1 H) ppm.
C₈H₇F₅O₂S (262.20): ber. C 36.65, H 2.69; gef. C 36.85, H 2.59.

### g) 2-Methyl-4-pentafluorsulfanyl-benzoylguanidin

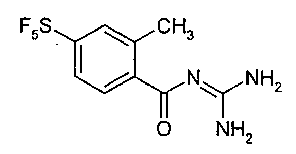

2-Methyl-4-pentafluorsulfanyl-benzoesäure (77,5 g, 295 mmol) wurde in Toluol (300 mL) suspendiert, mit Thionylchlorid (36 mL, 0,5 Mol) und 5 Tropfen DMF versetzt und das Gemisch 2 h unter Rühren am Rückfluß erhitzt. Anschließend wurde abgesaugt, das Filtrat im Vakuum eingedampft, der Rückstand 2 x in Toluol (je 100 mL) aufgenommen und jeweils im Vakuum eingedampft. Man erhielt 78,8 g des Säurechlorids als hellbraune Flüssigkeit, die ohne Reinigung weiterverwendet wurde. Zu einer Lösung von NaOH (84 g, 2,1 Mol) in Wasser (600 mL) wurde Guanidin-Hydrochlorid (172 g, 1,8 Mol) gegeben und das Gemisch auf-3 °C abgekühlt. Anschließend wurde unter Rühren und Eiskühlung innerhalb 1 h die Lösung des rohen Säurechlorids in Dichlormethan (600 mL) zugetropft. Es wurde weitere 30 min bei Raumtemperatur rühren gelassen und dann der ausgefallene Feststoff abgesaugt, mit Dichlormethan gewaschen und bei Raumtemperatur im Vakuum getrocknet. Man erhielt
74,3 g (87 %) 2-Methyl-4-pentafluorsulfanyl-benzoylguanidin als beige Kristalle,
Fp. 183-183,5 °C.
¹H-NMR 400 MHz, CD₃OD: δ = 2.51 (s, 3 H), 4.84 (bs, 5 H), 7.62 (m, 2 H), 7.65 (s, 1 H) ppm.
C₉H₁₀F₅N₃OS (303.26): ber. C 35.65, H 3.32, N 13.86; gef. C 35.69, H 3.18, N 14.04.

## Patentansprüche

1. Verfahren zur Herstellung von der Verbindungen der Formel I 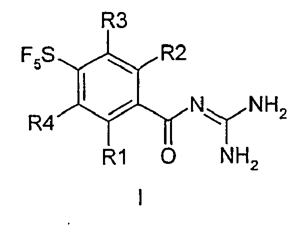 worin bedeuten:
R1 Wasserstoff, Alkyl mit 1, 2, 3 oder 4 C-Atomen, Alkoxy mit 1, 2, 3 oder 4 C-Atomen, NR10R11, -Oₚ-(CH₂)ₙ-(CF₂)ₒ-CF₃ oder -(SOₘ)_{q}-(CH₂)ᵣ(CF₂)ₛ-CF₃;
R10 und R11 unabhängig voneinander Wasserstoff, Alkyl mit 1, 2, 3 oder 4 C-Atomen oder -CH₂-CF₃;
m Null, 1 oder 2
n, o, p, q, r und s unabhängig voneinander Null oder 1;
R2 Wasserstoff, -(SOₕ)_{z}-(CH₂)ₖ -(CF₂)ₗ -CF₃, Alkyl mit 1, 2, 3, 4, 5 oder 6 C-Atomen, Cycloalkyl mit 3, 4, 5, 6, 7 oder 8 C-Atomen, in dem 1, 2, 3 oder 4 Wasserstoff-Atome durch Fluoratome ersetzt sein können;
h Null, 1 oder 2;
z Null oder 1;
k Null, 1, 2, 3 oder 4;
l Null oder 1;
oder
R2 -(CH₂)ₜ-Phenyl oder -O-Phenyl,
die unsubstituiert sind oder substituiert sind mit 1, 2 oder 3 Resten ausgewählt aus der Gruppe bestehend aus -Oᵤ-(CH₂)ᵥ-CF₃, Alkoxy mit 1, 2, 3 oder 4 C-Atomen, Alkyl mit 1, 2, 3 oder 4 C-Atomen und -SO₂CH₃;
t Null, 1, 2, 3 oder 4;
u Null oder 1;
v Null, 1, 2 oder 3;
oder
R2 -(CH₂)_{w}-Heteroaryl,
das unsubstituiert ist oder substituiert ist mit 1, 2 oder 3 Resten ausgewählt aus der Gruppe bestehend aus -Oₓ-(CH₂)_{y}-CF₃, Alkoxy mit 1, 2, 3 oder 4 C-Atomen und Alkyl mit 1, 2, 3 oder 4 C-Atomen, -SO₂CH₃;
w Null, 1, 2, 3 oder 4;
x Null oder 1;
y Null, 1,2 oder 3;
R3 und R4 unabhängig voneinander Wasserstoff oder F;
sowie deren Salze;
**dadurch gekennzeichnet, dass** 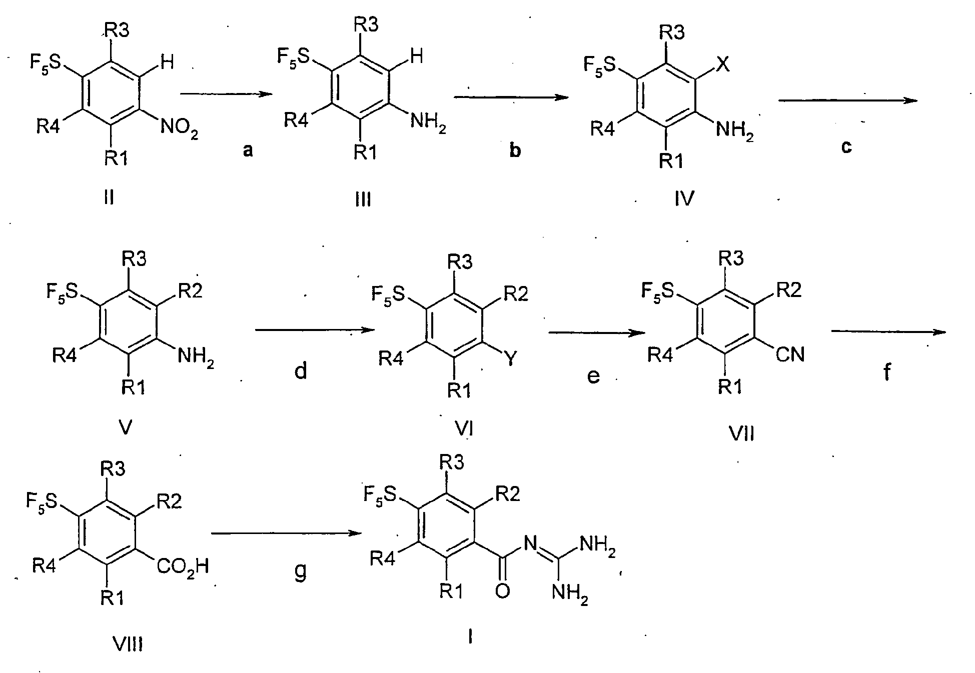
a) ein 4-Nitrophenyl-schwefelpentafluorid-Derivat der Formel II zum Amin der Formel III reduziert wird, und
b) die Verbindung der Formel III in ortho-Stellung zur Aminogruppe mit einem Halogenierungsmittel zur Verbindung der Formel IV halogeniert wird, und
c) in der Verbindung der Formel IV mit einem geeigneten Nukleophil oder einer Organoelementverbindung, beispielsweise einer Alkylborverbindung, gegebenenfalls unter Katalyse, der Halogen-Substituent durch einen Substituenten R2 ersetzt wird, und
d) in der Verbindung der Formel V die Aminofunktion durch einen Halogen-Substituenten ersetzt wird, und
e) in der Verbindung der Formel VI der Halogen-Substituent durch eine Nitrilfunktion ersetzt wird, und
f) die Nitrilfunktion der Verbindung der Formel VII zur Carbonsäure hydrolysiert wird, und
g) die Carbonsäure der Formel VIII in das Acylguanidin der Formel I überführt wird, wobei in den Verbindungen der Formeln II, III, IV, V, VI, VII und VIII
R1 bis R4 definiert sind wie in Formel I und
X und Y unabhängig voneinander F, Cl, Br oder I sind.

2. Verfahren gemäß Anspruch 1, wobei die Schritte a), b), c), d), e), f) und g) unabhängig voneinander kontinuierlich oder diskontinuierlich geführt werden.

3. Verfahren gemäß Anspruch 1 und/oder 2, worin R2 Wasserstoff ist und die Schritte b) und c) wegfallen.

4. Verbindungen der Formel X 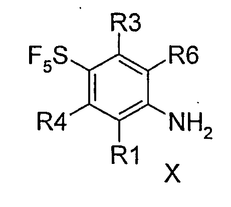 worin bedeuten
R1 Wasserstoff, Alkyl mit 1, 2, 3 oder 4 C-Atomen, Alkoxy mit 1, 2, 3 oder 4 C-Atomen, NR10R11, -Oₚ-(CH₂)ₙ-(CF₂)ₒ-CF₃ oder -(SOₘ)_{q}-(CH₂)ᵣ-(CF₂)ₛ-CF₃;
R10 und R11 unabhängig voneinander Wasserstoff, Alkyl mit 1, 2, 3 oder 4 C-Atomen oder -CH₂-CF₃;
m Null, 1 oder 2
n, o, p, q, r und s unabhängig voneinander Null oder 1;
R6 -(SOₕ)_{z}-(CH₂)ₖ -(CF₂)ₗ -CF₃, Alkyl mit 1, 2, 3, 4, 5 oder 6 C-Atomen, Cycloalkyl mit 3, 4, 5, 6, 7 oder 8 C-Atomen, in dem 1, 2, 3 oder 4 Wasserstoff-Atome durch Fluoratome ersetzt sein können;
h Null, 1 oder 2;
z Null oder 1;
k Null, 1, 2, 3 oder 4;
l Null oder 1;
oder
R6 -(CH₂)ₜ-Phenyl oder -O-Phenyl,
die unsubstituiert sind oder substituiert sind mit 1, 2 oder 3 Resten ausgewählt aus der Gruppe bestehend aus -Oᵤ-(CH₂)ᵥ-CF₃, Alkoxy mit 1, 2, 3 oder 4 C-Atomen, Alkyl mit 1, 2, 3 oder 4 C-Atomen und -SO₂CH3;
t Null, 1, 2, 3 oder 4;
u Null oder 1;
v Null, 1, 2 oder 3;
oder
R6 -(CH₂)_{w}-Heteroaryl,
das unsubstituiert ist oder substituiert ist mit 1, 2 oder 3 Resten ausgewählt aus der Gruppe bestehend aus -Oₓ-(CH₂)_{y}-CF₃, Alkoxy mit 1, 2, 3 oder 4 C-Atomen und Alkyl mit 1, 2, 3 oder 4 C-Atomen, -SO₂CH₃;
w Null, 1, 2, 3 oder 4;
x Null oder 1;
y Null, 1, 2 oder 3;
R3 und R4 unabhängig voneinander Wasserstoff oder F;
sowie deren Salze;

5. Verbindungen der Formel X und/oder deren pharmazeutisch verträgliche Salze nach Anspruch 4 zu Verwendung als Syntheseintermediat.

6. Verbindungen der Formel XI 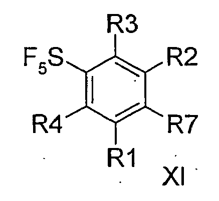 worin bedeuten
R1 Wasserstoff, Alkyl mit 1, 2, 3 oder 4 C-Atomen, Alkoxy mit 1, 2, 3 oder 4 C-Atomen, NR10R11, -Op-(CH₂)ₙ-(CF₂)ₒ-CF₃ oder-(SOₘ)q-(CH₂)ᵣ(CF₂)ₛ-CF₃;
R10 und R11 unabhängig voneinander Wasserstoff, Alkyl mit 1, 2, 3 oder 4 C-Atomen oder -CH₂-CF₃;
m Null, 1 oder 2
n, o, p, q, r und s unabhängig voneinander Null oder 1;
R2 Wasserstoff, F, Cl, Br, I, -(SOₕ)_{z}-(CH₂)ₖ -(CF₂)ₗ -CF₃ oder Alkyl mit 1, 2, 3, 4, 5 oder 6 C-Atomen;
h Null, 1 oder 2;
z Null oder 1;
k Null, 1, 2, 3 oder 4;
l Null oder 1;
R3 und R4 unabhängig voneinander Wasserstoff oder F;
R7 CN;
sowie deren Salze.

7. Verbindungen der Formel XI und/oder deren pharmazeutisch verträgliche Salze nach Anspruch 6 zu Verwendung als Syntheseintermediat.

## Claims

1. A process for preparing compounds of the formula I 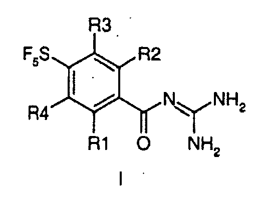
R1 hydrogen, alkyl having 1, 2, 3 or 4 carbon atoms, alkoxy having 1, 2, 3 or 4 carbon atoms, NR10R11, -Oₚ-(CH₂)ₙ-(CF₂)ₒ-CF₃ or -(SOₘ)_{q}-(CH₂)ᵣ-(CF₂)ₛ-CF₃;
R10 and R11 independently of one another hydrogen, alkyl having 1, 2, 3 or 4 carbon atoms or -CH₂-CF₃;
m zero, 1 or 2
n, o, p, q, r and s independently of one another zero or 1;
R2 hydrogen, -(SOₕ)_{z}-(CH₂)ₖ -(CF₂)ₗ -CF₃, alkyl having 1, 2, 3, 4, 5 or 6 carbon atoms, cycloalkyl having 3, 4, 5, 6, 7 or 8 carbon atoms, in which 1, 2, 3 or 4 hydrogen atoms may be replaced by fluorine atoms;
h zero, 1 or 2;
z zero or 1;
k zero, 1, 2, 3 or 4;
l zero or 1;
or
R2 -(CH₂)ₜ-phenyl or -O-phenyl, which are unsubstituted or substituted by 1, 2 or 3 radicals selected from the group consisting of -Oᵤ-(CH₂)ᵥ-CF₃, alkoxy having 1, 2, 3 or 4 carbon atoms, alkyl having 1, 2, 3 or 4 carbon atoms and -SO₂CH₃;
t zero, 1, 2, 3 or 4;
u zero or 1;
v zero, 1, 2 or 3;
or
R2 -(CH₂)_{w}-heteroaryl which is unsubstituted or substituted by 1, 2 or 3 radicals selected from the group consisting of -Oₓ-(CH₂)_{y}-CF₃, alkoxy having 1, 2, 3 or 4 carbon atoms and alkyl having 1, 2, 3 or 4 carbon atoms, -SO₂CH₃;
w zero, 1, 2, 3 or 4;
x zero or 1;
y zero, 1, 2 or 3;
R3 and R4, independently of one another hydrogen or F;
and the salts thereof;
which comprises 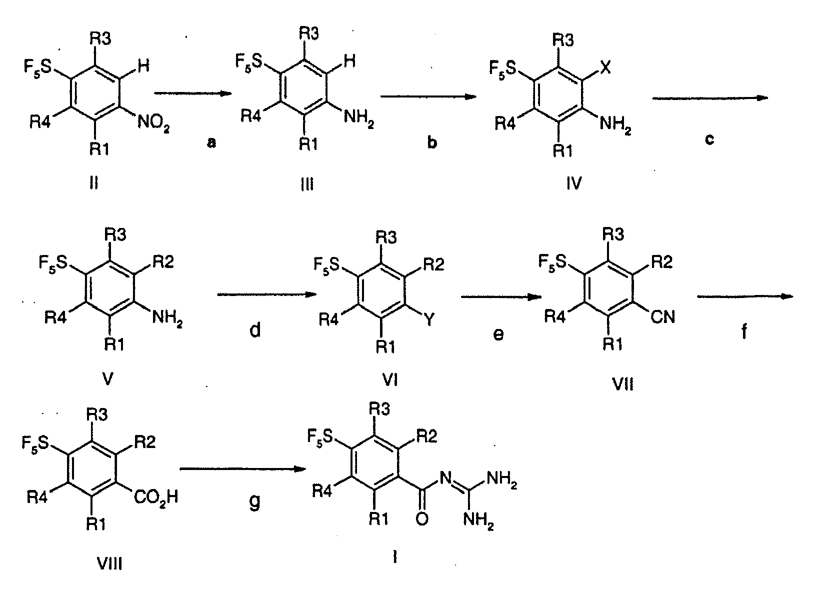
a) reducing a 4-nitrophenylsulfur pentafluoride derivative of the formula II to the amine of the formula III, and
b) halogenating the compound of the formula III in the ortho position to the amino group with a halogenating agent to give the compound of the formula IV, and
c) replacing the halogen substituent in the compound of the formula IV with a suitable nucleophile or an organoelement compound, for example an alkylboron compound, where appropriate with catalysis, by a substituent R2, and
d) replacing the amino function in the compound of the formula V by a halogen substituent, and
e) replacing the halogen substituent in the compound of the formula VI by a nitrile function, and
f) hydrolyzing the nitrile function of the compound of the formula VII to the carboxylic acid, and
g) converting the carboxylic acid of the formula VIII into the acylguanidine of the formula I,
where in the compounds of the formulae II, III, IV, V, VI, VII and VIII R1 to R4 are as defined in formula I and
X and Y are independently of one another F, Cl, Br or I.

2. The process as claimed in claim 1, where steps a), b), c), d), e), f) and g) are managed independently of one another continuously or discontinuously.

3. The process as claimed in claim 1 and/or 2, in which R2 is hydrogen, and steps b) and c) are omitted.

4. A compound of the formula X 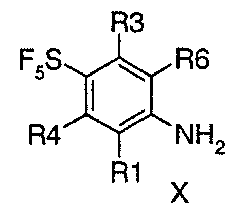 in which the meanings are:
R1 hydrogen, alkyl having 1, 2, 3 or 4 carbon atoms, alkoxy having 1, 2, 3 or 4 carbon atoms, NR10R11, -Oₚ-(CH₂)ₙ-(CF₂)ₒ-CF₃ or -(SOₘ)_{q}-(CH₂)ᵣ-(CF₂)ₛ-CF₃;
R10 and R11 independently of one another hydrogen, alkyl having 1, 2, 3 or 4 carbon atoms or -CH₂-CF₃;
m zero, 1 or 2
n, o, p, q, r and s independently of one another zero or 1;
R6 -(SOₕ)_{z}-(CH₂)ₖ -(CF₂)ₗ -CF₃, alkyl having 1, 2, 3, 4, 5 or 6 carbon atoms, cycloalkyl having 3, 4, 5, 6, 7 or 8 carbon atoms, in which 1, 2, 3 or 4 hydrogen atoms may be replaced by fluorine atoms;
h zero, 1 or 2;
z zero or 1;
k zero, 1, 2, 3 or 4;
l zero or 1;
or
R6 -(CH₂)ₜ-phenyl or -O-phenyl, which are unsubstituted or substituted by 1, 2 or 3 radicals selected from the group consisting of -Oᵤ-(CH₂)ᵥ-CF₃, alkoxy having 1, 2, 3 or 4 carbon atoms, alkyl having 1, 2, 3 or 4 carbon atoms and -SO₂CH₃;
t zero, 1, 2, 3 or 4;
u zero or 1;
v zero, 1, 2 or 3;
or
R6 -(CH₂)_{w}-heteroaryl which is unsubstituted or substituted by 1, 2 or 3 radicals selected from the group consisting of -Oₓ-(CH₂)_{y}-CF₃, alkoxy having 1, 2, 3 or 4 carbon atoms and alkyl having 1, 2, 3 or 4 carbon atoms, -SO₂CH₃;
w zero, 1, 2, 3 or 4;
x zero or 1;
y zero, 1, 2 or 3;
R3 and R4, independently of one another hydrogen or F;
and the salts thereof.

5. A compound of the formula X and/or the pharmaceutically suitable salts thereof as claimed in claim 4 for use as synthesis intermediate.

6. A compound of the formula XI 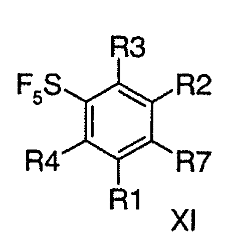 in which the meanings are:
R1 hydrogen, alkyl having 1, 2, 3 or 4 carbon atoms, alkoxy having 1, 2, 3 or 4 carbon atoms, NR10R11, -Oₚ-(CH₂)ₙ-(CF₂)ₒ-CF₃ or -(SOₘ)_{q}-(CH₂)ᵣ-(CF₂)ₛ-CF₃;
R10 and R11 independently of one another hydrogen, alkyl having 1, 2, 3 or 4 carbon atoms or -CH₂-CF₃;
m zero, 1 or 2
n, o, p, q, r and s independently of one another zero or 1;
R2 hydrogen, F, Cl, Br, I, -(SOₕ)_{z}-(CH₂)ₖ -(CF₂)ₗ -CF₃ or alkyl having 1, 2, 3, 4, 5 or 6 carbon atoms;
h zero, 1 or 2;
z zero or 1;
k zero, 1, 2, 3 or 4;
l zero or 1;
R3 and R4, independently of one another hydrogen or F;
R7 CN;
and the salts thereof.

7. A compound of the formula XI and/or the pharmaceutically suitable salts thereof as claimed in claim 6 for use as synthesis intermediate.

## Revendications

1. Procédé pour la préparation des composés de formule I 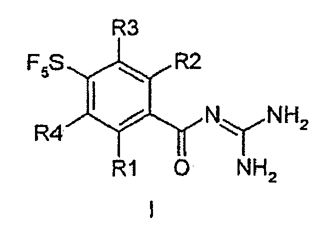 dans laquelle
R1 représente un atome d'hydrogène, un groupe alkyle ayant 1, 2, 3 ou 4 atomes de carbone, alcoxy ayant 1, 2, 3 ou 4 atomes de carbone, NR10R11, -Oₚ-(CH₂)ₙ-(CF₂)ₒ-CF₃ ou -(SOₘ)_{q}-(CH₂)ᵣ-(CF₂)ₛ-CF₃;
R10 et R11 représentent, indépendamment l'un de l'autre, un atome d'hydrogène, un groupe alkyle ayant 1, 2, 3 ou 4 atomes de carbone ou -CH₂-CF₃ ;
m est zéro, 1 ou 2 ;
n, o, p, q, r et s représentent, indépendamment les uns des autres, zéro ou 1 ;
R2 représente un atome d'hydrogène, -(SOₕ)_{z}-(CH₂)ₖ-(CF₂)ₗ-CF₃, un groupe alkyle ayant 1, 2, 3, 4, 5 ou 6 atomes de carbone, cycloalkyle ayant 3, 4, 5, 6, 7 ou 8 atomes de carbone, dans lequel 1, 2, 3 ou 4 atomes d'hydrogène peuvent être remplacés par des atomes de fluor ;
h est zéro, 1 ou 2 ;
z est zéro ou 1 ;
k est zéro, 1, 2, 3 ou 4 ;
l est zéro ou 1 ;
ou
R2 représente un groupe -(CH₂)ₜ-phényle ou O-phényle, qui est non substitué ou substitué par 1, 2 ou 3 radicaux choisis dans l'ensemble constitué par -Oᵤ-(CH₂)ᵥ-CF₃, alcoxy ayant 1, 2, 3 ou 4 atomes, alkyle ayant 1, 2, 3 ou 4 atomes de carbone et -SO₂CH₃;
t est zéro, 1, 2, 3 ou 4;
u est zéro ou 1;
v est zéro, 1, 2 ou 3;
ou
R2 représente un groupe -(CH₂)_{w}-hétéroaryle, qui est non substitué ou substitué par 1, 2 ou 3 radicaux choisis dans l'ensemble constitué par -Oₓ(CH₂)_{y}-CF₃, alcoxy ayant 1, 2, 3 ou 4 atomes de carbone et alkyle ayant 1, 2, 3 ou 4 atomes de carbone, -SO₂CH₃;
w est zéro, 1, 2, 3 ou 4 ;
x est zéro ou 1 ;
y est zéro, 1, 2 ou 3 ;
R3 et R4 représentent indépendamment l'un de l'autre, un atome d'hydrogène ou F ;
ainsi que de leurs sels ;
**caractérisé en ce que** 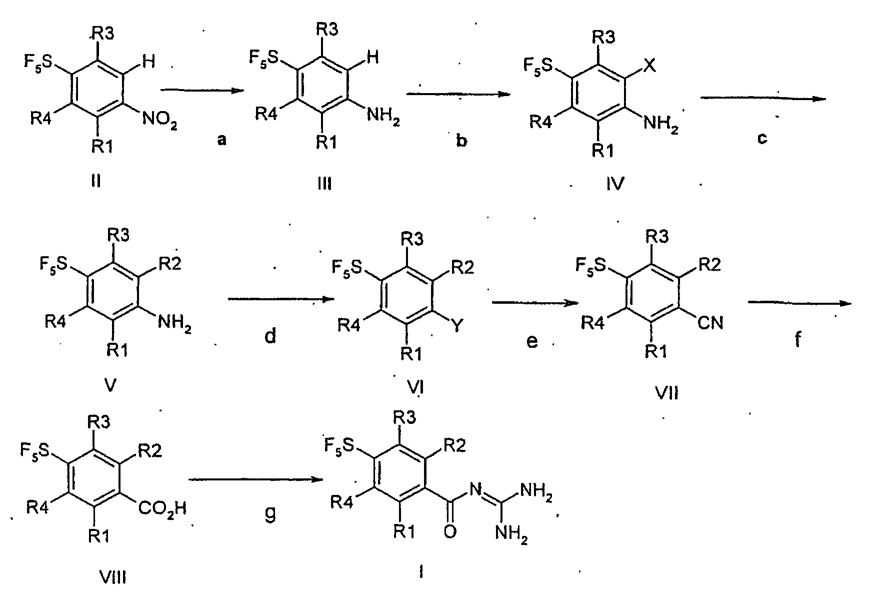
a) on réduit un dérivé de sulfopentafluorure de 4-nitrophényle de formule II en l'amine de formule III, et
b) on soumet à une halogénation le composé de formule III en position ortho par rapport au groupe amino, à l'aide d'un agent d'halogénation, pour aboutir au composé de formule IV, et
c) dans le composé de formule IV on remplace le substituant halogène par un substituant R2 au moyen d'un composé nucléophile ou d'un composé organo-élément, par exemple d'un composé alkylbore, éventuellement avec catalyse, et
d) dans le composé de formule V on remplace la fonction amino par un substituant halogène, et
e) dans le composé de formule VI on remplace le substituant halogène par une fonction nitrile, et
f) on soumet la fonction nitrile du composé de formule VII à une hydrolyse pour obtenir l'acide carboxylique, et
g) on convertit l'acide carboxylique de formule VIII en l'acylguanidine de formule I,
dans les composés de formules II, III, IV, V, VI, VII et VIII
R1 à R4 étant tels que définis dans la formule I et
X et Y représentant, indépendamment l'un de l'autre, F, Cl, Br ou I.

2. Procédé selon la revendication 1, dans lequel les étapes a), b), c), d), e), f) et g), indépendamment les unes des autres, sont effectuées en continu ou en mode discontinu.

3. Procédé selon la revendication 1 et/ou la revendication 2, dans lequel R2 est un atome d'hydrogène et les étapes b) et c) sont supprimées.

4. Composés de formule X 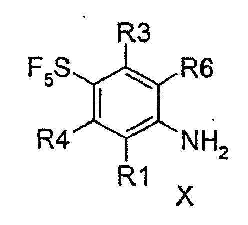 dans laquelle
R1 représente un atome d'hydrogène, un groupe alkyle ayant 1, 2, 3 ou 4 atomes de carbone, alcoxy ayant 1, 2, 3 ou 4 atomes de carbone, NR10R11, -Oₚ-(CH₂)ₙ-(CF₂)ₒ-CF₃ ou -(SOₘ)_{q}-(CH₂)ᵣ-(CF₂)ₛ-CF₃;
R10 et R11 représentent, indépendamment l'un de l'autre, un atome d'hydrogène, un groupe alkyle ayant 1, 2, 3 ou 4 atomes de carbone ou -CH₂-CF₃ ;
m est zéro, 1 ou 2 ;
n, o, p, q, r et représentent, indépendamment les uns des autres, zéro ou 1 ;
R6 représente -(SOₕ)_{z}-(CH₂)ₖ-(CF₂)ₗ-CF₃, un groupe alkyle ayant 1, 2, 3, 4, 5 ou 6 atomes de carbone, cycloalkyle ayant 3, 4, 5, 6, 7 ou 8 atomes de carbone, dans lequel 1, 2, 3 ou 4 atomes d'hydrogène peuvent être remplacés par des atomes de fluor ;
h est zéro, 1 ou 2 ;
z est zéro ou 1 ;
k est zéro, 1, 2, 3 ou 4 ;
l est zéro ou 1 ;
ou
R6 représente un groupe -(CH₂)ₜ-phényle ou O-phényle, qui est non substitué ou substitué par 1, 2 ou 3 radicaux choisis dans l'ensemble constitué par -Oᵤ-(CH₂)ᵥ-CF₃, alcoxy ayant 1, 2, 3 ou 4 atomes, alkyle ayant 1, 2, 3 ou 4 atomes de carbone et -SO₂CH₃ ;
t est zéro, 1, 2, 3 ou 4 ;
u est zéro ou 1 ;
v est zéro, 1, 2 ou 3 ;
ou
R6 représente un groupe -(CH₂)_{w}-hétéroaryle, qui est non substitué ou substitué par 1, 2 ou 3 radicaux choisis dans l'ensemble constitué par -Oₓ(CH₂)_{y}-CF₃, alcoxy ayant 1, 2, 3 ou 4 atomes de carbone et alkyle ayant 1, 2, 3 ou 4 atomes de carbone, -SO₂CH₃ ;
w est zéro, 1, 2, 3 ou 4 ;
x est zéro ou 1 ;
y est zéro, 1, 2 ou 3 ;
R3 et R4 représentent indépendamment l'un de l'autre, un atome d'hydrogène ou F ;
ainsi que leurs sels.

5. Composés de formule X et/ou sels pharmaceutiquement acceptables de tel composés, selon la revendication 4, pour utilisation en tant que produit intermédiaire de synthèse.

6. Composés de formule XI 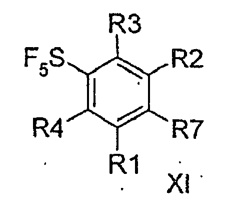 dans laquelle
R1 représente un atome d'hydrogène, un groupe alkyle ayant 1, 2, 3 ou 4 atomes de carbone, alcoxy ayant 1, 2, 3 ou 4 atomes de carbone, NR10R11, -Oₚ-(CH₂)ₙ-(CF₂)ₒ-CF₃ ou -(SOₘ)_{q}-(CH₂)ᵣ-(CF₂)ₛ-CF₃;
R10 et R11 représentent, indépendamment l'un de l'autre, un atome d'hydrogène, un groupe alkyle ayant 1, 2, 3 ou 4 atomes de carbone ou -CH₂-CF₃ ;
m est zéro, 1 ou 2 ;
n, o, p, q, r et s représentent, indépendamment les uns des autres, zéro ou 1 ;
R2 représente un atome d'hydrogène, F, CI, Br, I, -(SOₕ)_{z}-(CH₂)ₖ-(CF₂)ₗ-CF₃ ou un groupe alkyle ayant 1, 2, 3, 4, 5 ou 6 atomes de carbone ;
h est zéro, 1 ou 2 ;
z est zéro ou 1 ;
k est zéro, 1, 2, 3 ou 4 ;
l est zéro ou 1 ;
R3 et R4 représentent indépendamment l'un de l'autre, un atome d'hydrogène ou F ;
R7 représente CN ;
ainsi que leurs sels.

7. Composés de formule XI et/ou sels pharmaceutiquement acceptables de tels composés, selon la revendication 6, pour utilisation en tant que produit intermédiaire de synthèse.
